# EUROPEAN PATENT APPLICATION

(11) **EP 2 500 729 A1**
(43) Date of publication of application: **19.09.2012**
(21) Application number: 12171853.0
(22) Date of filing: 07.07.2003
(51) Int. Cl.: G01N 33/553, G01N 33/543, C12Q 1/68

(54) **Characterization of biochips containing self-assembled monolayers**

(30) Priority: 05.07.2002 US 393896 P
(62) Divisional of application: 03763298.1
(71) Applicant: The University of Chicago, Chicago, IL 60637 (US)
(72) Inventor: Mrksich, Milan, Chicago, IL 60614 (US); Su, Jing, Chicago, IL 60637 (US)
(74) Representative: Baldock, Sharon Claire

(57) **Abstract**

The present invention relates to a method of characterizing biochips with matrix-assisted laser desorption/ionization and time of flight mass spectrometry (MALDI-TOF MS).

## Description

This application is a continuation of U.S. provisional application 60/393,896, filed July 5, 2002, the contents of which is incorporated herein by reference in its entirety. This application claims priority to that provisional.

This work was supported in part by DARPA (N00173-01-1-G010).The government may have certain rights in this application.

### BACKGROUND OF THE INVENTION

Mass spectrometry (MS) is an important technique for characterizing the structures of surfaces and has several characteristics that are valuable in bioanalytical applications. In biochip and microarray applications, for example, MS offers the significant advantage that it does not require analytes to be labeled - either by direct attachment of fluorescent and radioactive labels or by binding of antibodies - and therefore offers greater flexibility in experiments. ^{[1-4]} Yet, MS remains a secondary option to the use of fluorescence and radioactivity for characterizing biochips, in part because many early studies have used home-built instrumentation and sophisticated protocols for data analysis. ^{[5-9]} Matrix-assisted laser desorption / ionization and time of flight mass spectrometry (MALDI-TOF MS), when combined with self-assembled monolayers (SAMs) that are tailored for biological applications, is well suited for characterizing biological activities as illustrated by the following examples that characterize the immobilization of ligands, the selective binding of proteins, and the enzymatic modification of immobilized molecules.

MALDI-TOF has been used for many years to identify peptides, proteins, carbohydrates and nucleic acids. In practice, aqueous samples are mixed with low molecular weight matrix molecules and dried on a metallic substrate prior to the MS analysis. Although MALDI MS is superior to other MS methods for analyzing biological complex, the presence of many components still leads to complicated spectra, which requires sophisticated analysis to identify specific analytes. Biochip applications, which rely on specific interactions of soluble and immobilized biomolecules, can avoid this limitation since only active components are retained on the substrate prior to MS analysis. [10-16]

### BRIEF SUMMARY OF THE INVENTION

The present invention provides SAMs that are engineered to give specific interactions with biomolecules, and therefore adds substantial flexibility to the use of MALDI in biochip applications. The SAMs of the present invention are inert to the non-specific adsorption of biomolecules

The SAMs of the present invention can have an overlaying layer with a plurality of openings, allowing multiple assays to be conducted thereon.

The SAMs of the present invention can be used in a variety of assays, including assays for biomolecular binding and enzymatic activity. The assay for enzymatic activity can be run with the enzyme ligand bound to the SAM. Alternatively, the enzyme ligand can be in the solution phase and after the assay is performed can be immobilized onto the SAM.

The present invention also provides kits for use in the assays described herein.

These and other inventions related to the SAMs of the present invention are described in detail below.

### BRIEF DESCRIPTION OF THE FIGURES

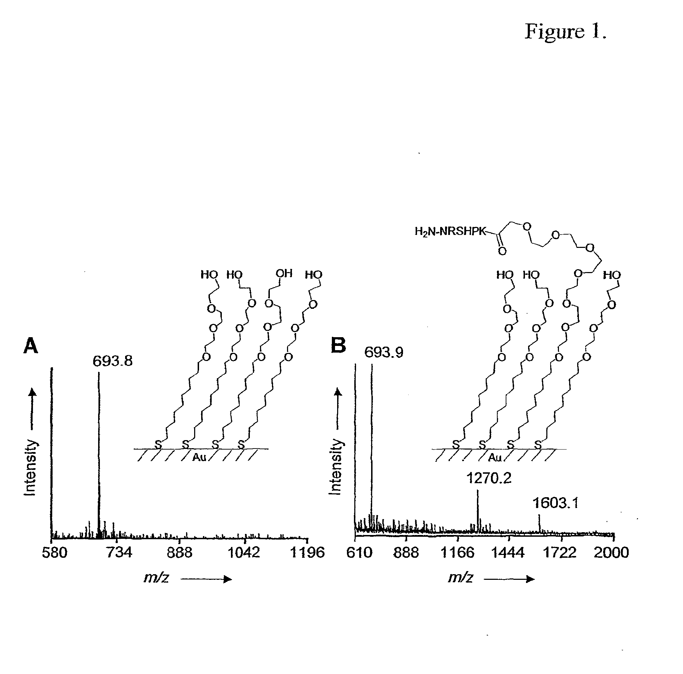

FIG 1 is a MALDI spectra of a monolayer presenting tri(ethylene glycol) groups (A) and a mixed monolayer presenting tri(ethylene glycol) groups and the peptide KPHSRN-NH₂ (B). The structure of each monolayer is shown above the spectrum. The principle peaks correspond to the symmetric glycol-terminated disulfide (*m*/*z* 693.8), the mixed peptide-terminated disulfide (*mlz* 1603.1) and the peptide-terminated alkanethiolate (*m*/*z* 1270.2).

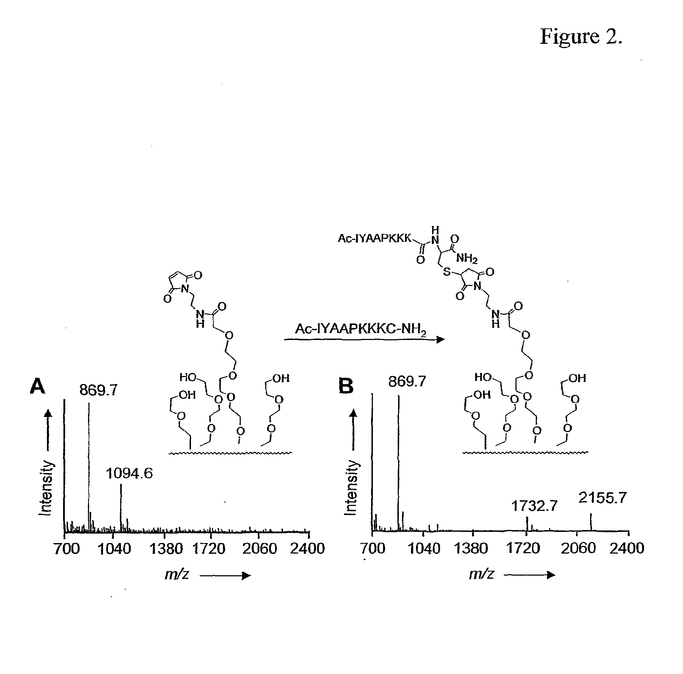

FIG 2(A) is a MALDI spectrum of a mixed monolayer presenting penta(ethylene glycol) groups and maleimide groups shows a peak for the mixed disulfide *(m*/*z* 1094.6). FIG 2(B) is the MALDI spectrum of the monolayer after treatment with the cysteine-terminated peptide *Ac*-IYAAPKKKC-*NH₂* shows mass peaks corresponding to immobilization of the peptide. The structure of each monolayer is shown above the spectrum.

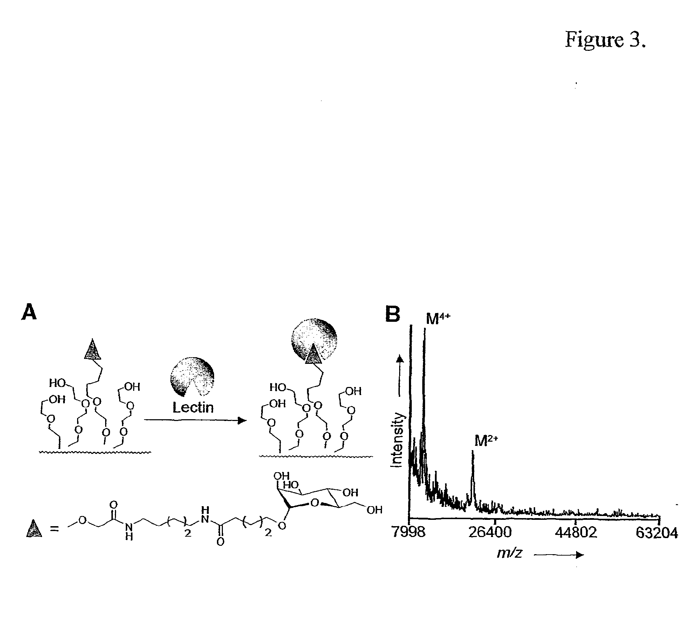

FIG 3(A) is a monolayer presenting the carbohydrate α-mannose was treated with an aqueous solution containing the lectin from *Vicia Sativa* (0.5 mg/ml in phosphate buffer, pH = 6.8), rinsed, and then analyzed by MALDI. FIG 3(B) is a spectra where the peaks at *m*/*z* 21.8 KD and 10.9 KD correspond to the double and tetra-ionized lectin and demonstrate that monolayers can be used to identify selective biomolecular binding interactions.

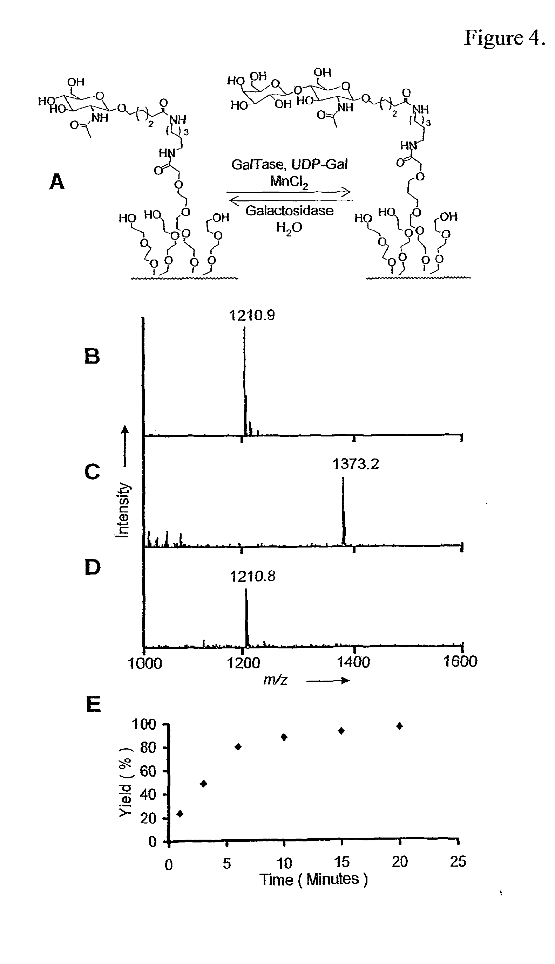

FIG 4(A) is a biochip analyzed by MALDI to reveal a peak at *mlz* 1210.9 for the mixed disulfide (B). After the monolayer was treated with the enzyme GalTase to introduce a terminal galactose residue, a MALDI spectrum revealed a new peak at *m*/*z* 1373.2 corresponding to the disaccharide product (C). Treatment of this monolayer with the enzyme galactosidase removed the terminal galactose, with regeneration of the GlcNAc group (D). MALDI was used to determine the time-dependence of the enzymatic galactosylation (E), demonstrating that this technique can provide kinetic information on biological activities.

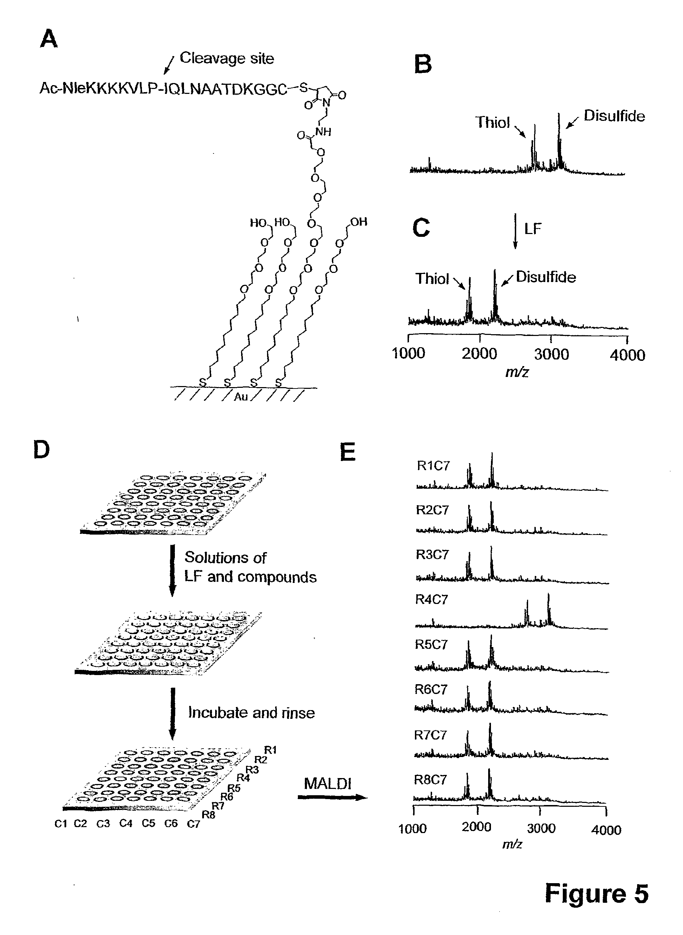

FIG 5(A) is a biochip presenting a peptide ligand that is enzymatically modified by the anthrax lethal factor protease. FIG 5(B) is a MALDI-TOF spectrum of this monolayer. FIG 5(C) is a MALDI-TOF spectrum of this monolayer after treatment with lethal factor protease. FIG 5(D) illustrates the procedure for applying multiple reaction mixtures to a single substrate and rinsing the reaction mixture from the substrate. FIG 5(E) shows representative mass spectra for spots representing distinct reaction mixtures. One of the eight spots shows a lack of peptide cleavage, denoting the presence of an inhibitor of LF in the reaction mixture.

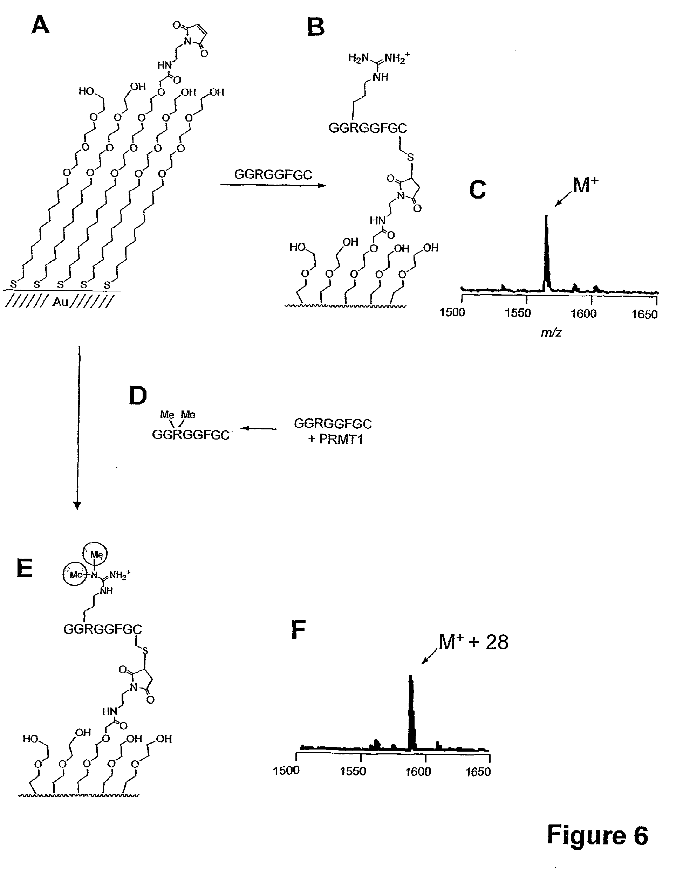

FIG 6(A) A monolayer presenting maleimide groups is used to immobilize a peptide which is enzymatically modified by the methyl transferase PRMT1 to yield a SAM presenting peptide (FIG 6B). FIG 6(C) A MALDI-TOF spectrum of this SAM. FIG 6(D) The peptide, dissolved in solution, is treated with the PRMT1 enzyme to yield a dimethylated peptide. FIG 6(E) The dimethylated peptide is immobilized to a SAM presenting maleimide groups. FIG 6(F) A MALDI-TOF spectrum of this SAM shows the presence of the enzymatically modified peptide.

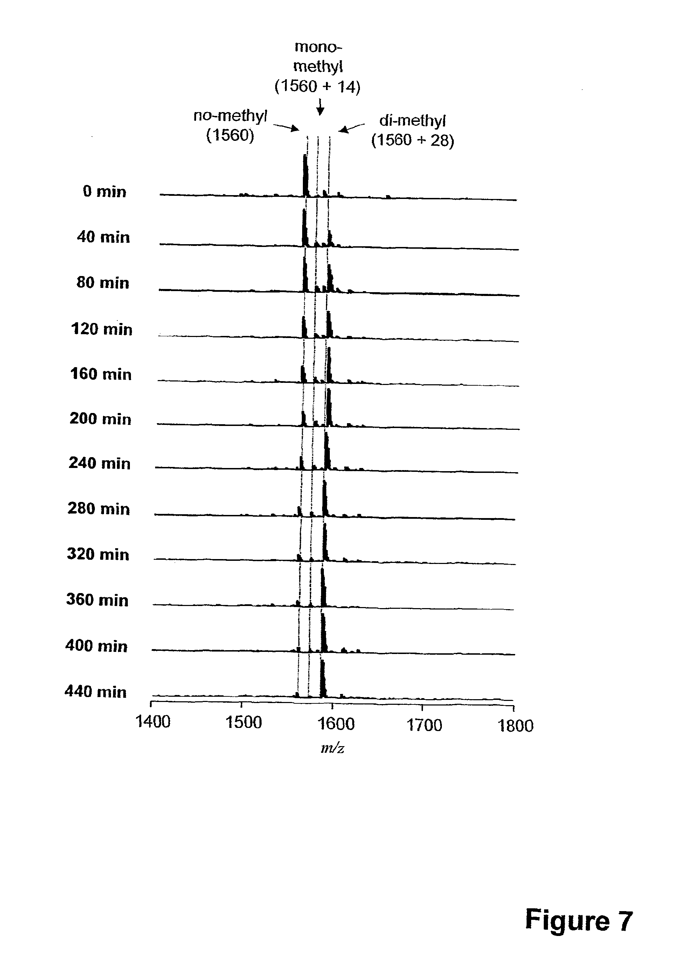

FIG 7 A time course for the enzymatic modification of a peptide by PRMT, as described in FIG 6. Each mass spectrum corresponds to a single time of reaction of the peptide and enzyme, and reveals the kinetic profile for the enzymatic reaction.

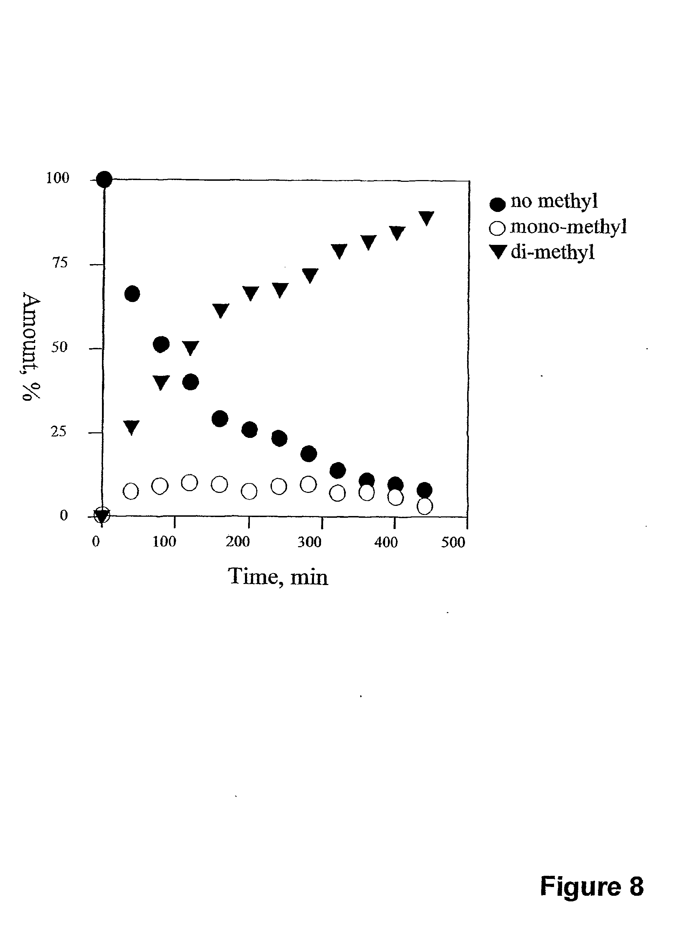

FIG 8 A quantified plot of the data shown in FIG 7. The unmodified peptide ligand is consumed during the reaction. The monomethylated peptide is present at a low fraction during the reaction. The dimethylated peptide product accumulates during the enzymatic reaction.

### SUMMARY OF THE INVENTION

### Biochips

The biochips of the present invention comprise self-assembled monolayers of alkanethiolates on a suitable metal surface (SAMs). The synthesis of SAMs is well known in the art (See, for example, U.S. published applications 20020119305 and 20020119054).

The metal surface is preferably silver, copper or gold or alloys thereof Preferably the metal surface is gold.

The surface may be on a substrate. The substrate may have the same composition as the surface (for example a gold surface on a gold plate), or the surface may be, for example, a film, foil, sheet, or plate, on a substrate having a different composition. The substrate may be any material, such as metal, metal oxide, glass, ceramic, plastic, or a natural material such as wood. Examples of substrates include glass, quartz, silicon, transparent plastic, aluminum, carbon, polyethylene, polypropylene, sepharose, agarose, dextran, polysytrene, polyacrylamide, a gel, and porous materials.

The surface material may be attached to the substrate by any of a variety of methods. For example, a film of the surface material may be applied to the substrate by sputtering or evaporation. If the surface material is a foil or sheet, it could be attached with an adhesive. Furthermore, the surface need not completely cover the substrate, but may cover only a portion of the substrate, or may form a pattern on the substrate. For example, sputtering the substrate, covering those portions of the substrate where no surface material is desired, may be used to pattern portions of the substrate. These patterns may include an array of regions containing, or missing, the surface material.

The methylene chain in the alkanethiolate can vary and is typically from 5 to 30 units, preferably 10-16. Alkanethiolates can be synthesized via reagents and reactions well know in the art, such as those described in "Advanced Organic Chemistry" J. March (Wiley & Sons, 1994); and "Organic Chemistry" 4th ed., Morrison and Boyd (Allyn and Bacon, Inc., 1983). The SAMs of the invention can be formed from alkanethiols or dialkyldisulfides. In both cases, the sulfur atom coordinates to the metal. The polymethylene chain is in an extended conformation. The SAMS can be prepared by immersing the metal in solutions containing the alkanethiol or dialkyldisulfides. The density of alkanethiolates on the metal surface is about 10¹⁰ molecules/cm².

SAMs which are inert to the non-specific adsorption of biomolecules can be formed from a variety of functionalized alkanethiols, including those that are terminated in the oligo(ethylene glycol) group, the mannitol group, the oligo(propylene sulfoxide) group and others. Syntheses of functionalized alkanethiols are described, for example, in U.S. published applications 20020119305 and 20020119054). "Non-specific adsorption" refers to the adsorption of a protein onto a surface by an interaction other than a ligand / receptor interaction. The inertness of the SAMs maximizes the activity of the immobilized ligand and reduces false signals due to non-specifc interactions. ^{[17-22]}

When the alkanethiol is terminated with oligo(ethylene glycol) groups, the oligo(ethylene glycol) oligomer preferably contains 3 to 7 units. When the alkanethiol is terminated with oligo(propylene sulfoxide) group, the oligo(propylene sulfoxide) oligomer preferably contains 3 units.

In applications in which the ligand is immobilized onto the SAM, the ligand can be immobilized using a variety of coupling strategies, including cycloaddition reactions, condensation reactions (such as those between amines and carboxylic acids, amines and aldehydes, etc.), reactions between thiols and maleimide, reactions between thiols and α-haloketones, reactions between thiols and activated sulfides (to yield a disulfide linked ligand), etc. Alternatively, ligands can be immobilized onto the SAM via a reaction of a protein with a ligand (e.g. GST binding glutathione) or with an irreversible ligand, such as disclosed in U.S. published patent application 20030119054.

Suitable ligands which can be immobilized onto the surface of the SAMs of the present invention include biomolecules (such as peptides, proteins, carbohydrates, oligosaccharides, oligonucleotides, antibodies, Fab fragments, etc.) or non-natural compounds (such as small molecules, chelating molecules, drugs, peptidomimetics, nucleic acid analogs, antibody mimics, imprinted polymers, etc.).

The SAMs of the present invention present ligands at low densities (≤ 20%). From between about 0.001% to 20%, preferably from between about 0.5 to 5%, of the alkanethiols on the SAM present the ligand. The remaining alkanethiol are terminated as described above in order to render the metal surface inert to non-specific adsorption.

### MALDI-TOF MS

Matrix-assisted laser desorption / ionization and time of flight mass spectrometry (MALDI-TOF MS) can be used to characterize SAMs. One example is provided below.

In general, a SAM is provided. Optionally, a matrix can be applied to the SAM, and preferably is. Suitable matrices which can be used in this invention are known in the art, and include, for example, substituted benzoic acids. One preferred matrix is 2,5-dihydroxyl benzoic acid. The matrix can be applied by delivering a solution containing the matrix to the metal surface. The concentration of the matrix can vary; typically it is between 1 and 50 mg/mL. The solvent can vary; typically it is acetonitrile or an alcohol (such as ethanol, methanol, isopropanol, etc.).

Figure 1 shows a spectrum of a monolayer prepared from tri(ethylene glycol)-terminated alkanethiol and shows a single intense peak at *mlz* 693.8. This mass corresponds to the sodium adduct of the symmetric disulfide (Figure 1A), ^{[24]} and agrees with previous reports that predominantly observe molecular ions of disulfides from alkanethiolate SAMs. ^{[7, 9]} A MS spectrum of a monolayer presenting a mixture of tri(ethylene glycol) groups and the hexapeptide KPHSRN-*NH₂* (in a ratio of 19:1) reveals an intense peak that corresponds to the symmetric disulfide terminated with glycol groups (*m*/*z* 693.9) and a second peak for the mixed disulfide presenting one glycol group and one peptide (*mlz* 1603.1) (Figure 1B). ^{[25]} The small peak at *mlz* 1270.2 is due to the peptide-terminated alkanethiol.

MALDI MS can also be applied to characterizing the immobilization of biomolecules to SAMs. Figure 2A shows a MS spectrum for a SAM presenting maleimide and penta(ethylene glycol) groups (in a ratio of 1:4). The spectrum shows the expected peaks for the symmetric glycol-substituted disulfide (*mlz* 869.7) and for the mixed disulfide containing one maleimide group (*m*/*z* 1094.6). The monolayer was treated with an aqueous solution containing the cysteine-terminated peptide *Ac*-IYAAPKKKC-*NH₂* (2 mM) for 2 hours, rinsed and then analyzed by MS. The absence of the peak at *m*/*z* 1094.6 shows that the maleimide group had reacted under these conditions and the two new peaks at *mlz* 1732.7 and 2155.7 represent the products resulting from Michael addition of the cysteine-terminated peptide with the maleimide group (Figure 2B). These peaks represent, respectively, the peptide-terminated alkanethiol and the mixed disulfide.

The following examples demonstrate that the combination of MALDI-TOF and glycol-terminated SAMs is well suited for the types of assays that are implemented with biochips. In the first example, a monolayer presenting the carbohydrate α-mannose and tri(ethylene glycol) groups (in a ratio of 1: 4) was treated with a solution of the lectin from *Vicia Sativa* (molecular weight ∼43 KD, 0.5 mg/ml in phosphate buffer, pH = 6.8) for 30 minutes and then rinsed with distilled water (Figure 3A). A solution of sinapinic acid (a common matrix in MALDI) in acetonitriie-0.1% trifluoroacetic acid-H₂O (10 mg/ml) was applied to the monolayer and allowed to evaporate prior to MALDI analysis. The spectrum in Figure 3B reveals peaks corresponding to the multiply ionized lectin, demonstrating that MALDI can directly observe proteins that have bound to ligands immobilized to monolayers. Identical experiments with monolayers presenting either β-*N-*acetylglucosamine (β-GlcNAc), which is not a substrate for this lectin, or monolayers presenting only glycol groups gave no peaks in this mass range, demonstrating that the protein association with the monolayer was biospecific.

In the second example, MALDI was used to characterize the enzymatic modification of an immobilized ligand (Figure 4A). A monolayer presenting the carbohydrate β-GlcNAc and tri(ethylene glycol) groups (in a ratio of 1:4) was prepared. MALDI showed a single intense peak at *mlz* 1210.9, corresponding to the mixed disulfide containing a single GlcNAc group (Figure 4B). This monolayer was then treated with a HEPES buffer (50 mM, pH = 7.5) containing β-1,4-galactosyltransferase (GalTase, 250 mU/ml), MnCl₂ (10 mM) and uridine diphosphogalactose (UDP-Gal, 20 µM) for 1 hour at 20°C and then rinsed. Analysis by MALDI showed a single intense peak at *mlz* 1373.2, corresponding to the disaccharide product *N*-acetyllactosamine (LacNAc) that results from enzymatic galactosylation of GlcNAc (Figure 4C). The absence of a peak at *m*/*z* 1210.9 demonstrates that the enzymatic reaction had gone to completion. This substrate was treated with a solution containing the enzyme galactosidase (25 u/ml), MgCl₂ (1 mM), KCl (10 mM) and β-mercaptoethanol (50 mM) in phosphate buffer (pH = 7.0) for 8 hours at 37°C. MALDI revealed that the LacNAc was enzymatically converted to GlcNAc in quantitative yield (Figure 4D). Control experiments show that treatment of monolayers presenting α-mannose with either GalTase or galactosidase had no effect, again demonstrating the specificity intrinsic to this class of SAMs.

In a final example, the combination of MALDI-TOF MS and SAMs was used to demonstrate that kinetic data can be provided for biological interactions on chips. The time-dependence of the interfacial galactosylation was investigated by treating identical SAMs presenting β-GlcNAc with GalTase as described above for periods of time ranging from 0 to 20 minutes. The monolayers were each rinsed, dried, and analyzed by MALDI. We calculated the yield for enzymatic conversion on each chip by taking a ratio of the peak height for LacNAc relative to the combined peak heights for LacNAc and GlcNAc (yield = H_{L}/[H_{L}+H_{G}]). ^{[26]} Figure 4E demonstrates that the yield increased smoothly with time and reached a plateau at complete conversion. This result indicates that MALDI has the characteristics required for kinetic analysis of interfacial reactions.

The most significant result of this work is that a commercial instrument for MALDI-TOF MS, when combined with self-assembled monolayers engineered for bioanalytical applications, is a very effective technique for characterizing biological activities, at interfaces. This finding can be exploited for a range of purposes, but in particular for examining biochips. The recent development of strategies that use self assembled monolayers for the preparation of peptide, protein and carbohydrate arrays makes this technique immediately applicable. ^{[22, 27, 28]} The use of MALDI in these applications is significant because this method can identify unexpected biological activities while current methods for characterizing biochips require preliminary knowledge of the activity to be identified. Fluorescence detection of antibodies that bind to arrays, for example, will only identify activities that affect the presence of antigen. MS, by contrast, will identify any change in mass at the interface-whether due to binding of a protein or modification by an enzyme-and hence can discover unanticipated activities. These properties, together with the widespread availability of the commercial instruments, can be used to make MALDI an extensive and dominant technique for application in bioanalytical and surface chemistry.

### Assays

The biochips of the present invention can be used to assay for a variety of biomolecules using MALDI-TOF MS.

The biochips of the present invention can also be used in high throughput screens (HTS). In HTS for protein binding, a plurality of biochips presenting different ligands can be used. Alternatively, a biochip presenting different ligands can be used. Preferably, a biochip presenting different ligands in isolated regions on the biochip is used.

In HTS for enzymatic activity, it is preferable to use a biochip presenting a ligand in isolated regions on the biochip. In this embodiment, the enzyme and candidate inhibitor are contacted with discrete regions of the biochip.

Biochips with physically separated regions are described below.

Matrices which can be used in the assay of the present invention are the same matrices described above for MALDI-TOF MS.

### Biomolecular Binding Assays

The method of the present invention involves providing a SAM that is capable of covalently binding a biomolecule, contacting the SAM with a sample which may contain the biomolecule, rinsing the SAM, optionally applying a matrix, and analyzing the matrix with MALDI-TOF MS.

In general, the SAM of the present invention presents a ligand that specifically binds the biomolecule (such as those described above, preferably proteins). "Specific binding" refers to the association of a ligand with a biomolecule to form an intermolecular complex. In one embodiment, the monolayer can present a carbohydrate that binds to a protein (such as a lectin) as exemplified below. Other interactions include antigen/antibody, antigen/Fab fragment, peptide/protein, non-natural molecule protein, oligonucleotide/oligonucleotide, protein/oligonucleotide, phosphopeptide/protein, phosphopeptide/antibody.

Suitable samples which can be assayed using the present invention can vary. Exemplary samples include solutions which may contain a biomolecule, such as cell lysates, blood samples, tissue samples, chromatography fractions, reaction mixtures, etc. The volume of the sample applied to the biochip will vary depending on the binding affinity and association rate constant of the biomolecule for the ligand presented by the SAM. Typically, ligand/biomolecule pairs having equilibrium association constants of about 10⁴ M⁻¹ or greater can be detected.

### Enzyme Activity Assays

The method of the present invention involves providing a SAM that presents a ligand capable of undergoing an enzymatic modification, contacting the SAM with a sample containing an enzyme, rinsing the SAM, optionally applying a matrix, and analyzing the matrix with MALDI-TOF MS.

In general, the SAM of the present invention presents a ligand capable of undergoing an enzymatic modification, such as a protein, peptide, carbohydrate, metabolite, non-natural molecule, lipid, etc. Examples of enzymatic modifications include an modification that results in a change in the mass of the ligand immobilized to the SAM. Exemplary modifications include acyl transfer, proteolysis, phosphorylation, glycosylation, oxidations, reductions, dehydrogenations, hydroxylations, eliminations, decarboxylations, carboxylations, aldol condensations, Claisen condensations, methylations, demethylations, etc.

The enzyme is contacted with the SAM presenting the ligand for a time sufficient to allow the enzyme to modify the ligand. Times may vary. Indeed, an analysis of the time dependent yields of the modified ligand can provide kinetic information on enzyme activity. Other reaction conditions can also vary, including temperature, solvent, buffer, etc.

The assays of the present invention can also be used to study inhibitors of the enzyme. In this embodiment, the SAM presenting the ligand would be contacted with the enzyme and the putative inhibitor.

### Solution phase enzymatic assays

In applications where it is desirable to first react the ligand and enzyme in solution (versus an immobilized ligand), it is possible to use the SAMs of the present invention. In a first embodiment (exemplified below), the enzyme and ligand are first contacted in solution and then applied to a SAM presenting a group that can selectively immobilize the ligand (in modified or unmodified form or mixtures). For example, a SAM presenting a maleimide is contacted with a solution containing a cysteine terminated peptide (where the peptide had previously been enzymatically modified in solution), the SAM rinsed to remove non-immobilized reactants, and analyzed by MALDI-TOF.

In a second embodiment, the SAM is functionalized with a group which can be activated/deactivated. In this embodiment, the enzyme and ligand are first contacted in solution and then applied to a SAM presenting a group that can be activated. Upon activation, the SAM immobilizes the ligand (in modified or unmodified form or mixtures). The SAM can be activated electrically, photolytically, chemically, enzymatically, thermally, etc. For example, a SAM presenting a hydroquinone group can be used to immobilize peptides modified with a diene. Upon activation with an electrical potential, the hydroquionone converts to benzoquinone which then selectively reacts with the diene in the peptide to immobilize the peptide (See, for example, M. N. Yousaf, B. T. Houseman and M. Mrksich Angevv. Chem. Int. Ed., 2001, 40, 1093-1096).

### Biochips with discrete regions

The biochips of the present invention can optionally include an overlaying layer with one or more holes. This layer, when present, allows discrete regions of the biochip to be modified. For example, in HTS for enzyme inhibitors, a SAM presenting a single ligand and an overlaying layer with 96 holes, so that it resembles a microtiter plate. Each "well" (formed by a hole in the overlaying layer) could be contacted with a solution of enzyme and a different putative inhibitor. Following modification, the overlaying layer could be removed so that the SAM could be assayed using the MALDI-TOF techniques described above.

The overlaying layer can be composed of a variety of materials, including plastics, elastomers, composites, etc. The overlaying layer can be attached to the SAM through direct physical contact or via an adhesive layer.

### EXAMPLES

The following examples describe the uses for ligand-modified self-assembled monolayers.

### Example 1

### Protein Binding of Con A to Mannose

The following example demonstrates that the combination of MALDI-TOF and SAMs presenting ligands and that are otherwise inert is well suited for assays that use biochips to identify proteins in a sample. The strategy uses a SAM presenting a ligand that selectively binds to a protein in order to selectively bind the protein from a sample. Following rinsing of the chip to remove the solution and species that are not bound by the SAM, the SAM is analyzed by MALDI-TOF to identify the bound protein. This strategy can be applied to a broad range of analytes for which a selective ligand is available. In one example, a SAM presenting the carbohydrate α-mannose and tri(ethylene glycol) groups (in a ratio of 1:4) was treated with a solution of the lectin from *Vicia Sativa* (molecular weight ∼43 KD, 0.5 mg/ml in phosphate buffer, pH = 6.8) for 30 minutes and then rinsed with distilled water (Figure 3A). A solution of sinapinic acid (a common matrix in MALDI) in acetonitrile-0.1% trifluoroacetic acid-H₂O (10 mg/ml) was applied to the SAM and allowed to evaporate prior to MALDI analysis. The spectrum in Figure 3B reveals peaks corresponding to the multiply ionized lectin, demonstrating that MALDI can directly observe proteins that have bound to ligands immobilized to SAMs. Identical experiments with SAMs presenting either β*-N-*acetylglucosamine (β-GlcNAc), which is not a ligand for this lectin, or SAMs presenting only glycol groups gave no peaks in this mass range, demonstrating that the protein association with the SAM was biospecific.

### Example 2 - Carbohydrate Modifying Enzyme

In another example, MALDI-TOF was used to characterize the enzymatic modification of an immobilized ligand (Figure 4A). A SAM presenting the carbohydrate β-GlcNAc and tri(ethylene glycol) groups (in a ratio of 1:4) was prepared. Analysis of this SAM by MALDI-TOF showed a single intense peak at *m*/*z* 1210.9, corresponding to the mixed disulfide containing a single GlcNAc group (Figure 4B). This SAM was then treated with a HEPES buffer (50 mM, pH = 7.5) containing β-1,4-galactosyltransferase (GalTase. 250 mU/ml), MnCl₂ (10 mM) and uridine diphosphogalactose (UDP-Gal, 20 µM) for 1 hour at 20°C and then rinsed. Analysis by MALDI-TOF showed a single intense peak at *mlz* 1373.2, corresponding to the disaccharide product N-acetyllactosamine (LacNAc) that results from enzymatic galactosylation of GlcNAc (Figure 4C). The absence of a peak at *m*/*z* 1210.9 demonstrates that the enzymatic reaction had gone to completion. This SAM was then treated with a solution containing the enzyme galactosidase (25 U/ml), MgCl₂ (1 mM), KCI (10 mM) and β-mercaptoethanol (50 mM) in phosphate buffer (pH = 7.0) for 8 hours at 37°C. MALDI revealed that the LacNAc was enzymatically converted to GlcNAc in quantitative yield (Figure 4D). A time course of this reaction shows that MALDI-TOF provides kinetic information (Figure 4E). Control experiments show that treatment of SAMs presenting α-mannose with either GalTase or galactosidase had no effect, again demonstrating the specificity intrinsic to this class of SAMs.

### Example 3 - Chemical Screening

The ability to conduct enzymatic activity assays without the need to use chromatography or other purification strategies to prepare the sample for analysis by MALDI-TOF makes this technique well-suited for chemical screening programs. Here, chemical screening refers to the evaluation of many compounds (from 100 to 10,000,000) in a biological assay to identify compounds that act as agonists or antagonists for specific proteins or enzymes. One example applied this strategy to identify antagonists of the anthrax lethal factor (LF) protease. The assay for LF uses a SAM that presents a peptide against a background of tri(ethylene glycol) groups (Figure 5A) The SAMs were prepared by immersing gold coated glass cover slips in an ethanolic solution containing a maleimide-terminated disulfide and a tri(ethylene glycol)-terminated disulfide to generate maleimide functionalized SAMs, using methods reported in a recent publication (B. T. Houseman, E. S. Gawalt and M. Mrksich Langmuir, 2003,19,1522-1531). A cysteine-terminated peptide ligand for LF was immobilized by spotting the peptide solution (1 mM in pH 7.0 Tris Buffer) on the monolayer for 30 minutes at 37 °C in a humidified chamber. (13). The peptide is a ligand that is enzymatically modified by LF and is cleaved by the enzyme at the proline residue (15). The glycol groups serve to prevent non-specific adsorption of protein to the surface and ensure that all the peptides remain available for interaction with the enzyme (16, 17). Analysis of the substrate with a commercial instrument for MALDI-TOF showed two mass to charge peaks, corresponding to the peptide-terminated alkanethiol (sodium adduct, m/z = 2794) and the disulfide substituted with one peptide and one glycol group (sodium adduct, m/z = 3130) (Figure 5B) (*18*). The well-defined surface chemistry and the lack of fragmentation of molecules are both important to giving clear and easily interpreted spectra. When this substrate was treated with LF and rinsed, MALDI-TOF revealed that these two peaks were absent and gave rise to two new peaks corresponding to proteolysis of the peptide (sodium adducts for both peaks, m/z = 1859 and 2195)(Figure 5C). LF was purchased from List Biological Laboratories and stored as recommended by the provider. The assay buffer for enzyme reactions was 25 mM HEPES at pH 7.0 containing 10 mM NaCI, 5 mM MgCl₂, 50 µM CaCl₂, and 50 µM ZnCl₂.).

The assay described above was applied to screen a library of 10,000 molecules to identify inhibitors of LF (Figure 5D) (20). Cocktail solutions containing LF (200 nM) and eight compounds from the library (with each compound present at approximately 10 µM concentration) in the same assay buffer described above were first prepared. To prepare the plate for MALDI-TOF analysis, a glass plate was machined to give a 10 by 10 array of circular grooves (2 mm in diameter), and then modified by evaporating a gold film on the plate, and assembling a SAM presenting the maleimide groups which were then derivatized with the peptide. The mixtures containing LF and either compounds were arrayed onto the plate within the circular grooves-which served to control the spreading of the drop to a constant area-and then incubated for 10 minutes at 37 °C in the humidified chamber. The SAMs were rinsed with portions of distilled water, dilute hydrochloric acid (1 µM), distilled water, and absolute ethanol. The SAMs were then treated with matrix (5 % 2,4,6-trihydroxyacetophenone in methanol) and analyzed by MALDI-TOF on a Voyager-DE Biospectroscopy mass spectrometer to obtain a mass spectrum for each circular region. Figure 5E shows representative MS data for eight of the mixtures (a total of 1250 mixtures were assayed). The majority of spots on the SAM show complete cleavage of the immobilized peptides. Eleven of the spots (approximately 1%) showed no cleavage or incomplete cleavage of the peptide, indicating the presence of an inhibitor in the cocktail. The assay was repeated with each of the eighty-eight compounds and found one compound, DS-998, that completely blocked LF activity at 10 µM concentration.

### Example 4 - Pull-Down with PMRT

In certain cases, it is not feasible to use an immobilized substrate to test the activity of an enzyme. One reason is that immobilization of the substrate to a solid phase may compromise its activity for the enzyme. A second reason is that the enzyme may act on the immobilized substrate with different kinetics than it does on the corresponding soluble substrate- For these reasons, it is important to have assay formats that allow the enzyme activity assay to be conducted in solution, with a freely soluble substrate, and then to transfer the substrate (whether or not it has been modified by the enzyme) to a SAM so that it can be analyzed by MALDI-TOF. Further, when the assay solution is applied to the SAM, it is important that the substrate be selectively and efficiently immobilized to the surface so that purification of the substrate from the enzyme reaction mixture can be avoided. A variety of selective immobilization schemes are available for immobilizing the desired substrate from the mixture, including the use of the cycloaddition reactions, the reaction of thiols with maleimide, the reaction of cutinase with phosphonate ligands, and many others.

The following example illustrate this strategy with PRMTI, which is the predominant type I protein arginine methyltransferase that transfers methyl groups from S-adenosyl-L-methionine (AdoMet) to proteins. Most PRMT1 substrates contain glycine- and arginine-rich sequences that include multiple arginines (X. Zhang and X. Cheng, Structure, 11, 509-520, 2003**)**.

A GST fusion of PRMT1 (GST-PRMT1) was expressed from plasmid pGEX-2T-PRMT1 as described in (W.-J. Lin et. al. J. Biol. Chem., 271 (25), 15034-15044, 1996**,** J. Tang et al. J. Biol. Chern., 275 (11), 7723-7730, 2000**).** The peptide GGRGGFGC was synthesized using conventional FMOC-solid phase synthesis and used as a substrate for the enzyme. This peptide was immobilized to a SAM presenting maleimide groups and characterized by MALDI-TOF to show the immobilization of peptide (Figures 6A-C). The immobilized peptides were not efficiently modified by the PRMT1 enzyme. Instead, the assay was conducted in solution followed by selective immobilization of the peptide ligand to a SAM presenting maleimide groups.

The maleimide-terminated SAMs were formed as described in the literature (B. T. Houseman, E. S. Gawalt and M. Mrksich Langmuir, 2003, 19, 1522-1531). A solution (5 µl) containing the GST-PRMT1 enzyme (at 20 µM concentration) was mixed with a 3 µl solution containing AdoMet (purchases from Sigma, total concentration of 5 mM) and incubated at 37°C for 1 minute before the peptide ligand was added. The enzyme reaction was initiated by addition of a solution containing the peptide ligand at pH 8.0 in Tris buffer to give a final volume of 10 µl (Figure 6D-F). The final concentrations of GST-PRMTl, AdoMet, and the peptide ligand were 10 µM, 1.5 mM, and 0.5 mM, respectively, in 10 µl of the reaction solution. The reaction mixture was incubated at 37 °C for variable times. To obtain a kinetic profile of the reaction, 0.6 µl of the reaction was removed at each of several time points, quenched and then transferred onto the maleimide-presenting SAM (within circles, 2 mm in diameter) at each time point and incubated at 37 °C for 20 minutes for peptide immobilization. The biochip was rinsed with distilled water, dilute acid (10 µM HCl), distilled water and ethanol. This procedure was repeated for each time point. The SAMs were then treated with matrix (5 % 2,4,6-trihydroxyacetophenone in methanol) and analyzed by MALDI-TOF MS to obtain a mass spectrum for each circle (Figure 7). The amount of product was quantitated from the intensities of peaks for the unmodified peptide ligand and the methylated peptide ligand, and plotted to provide kinetic information on the enzymatic reaction (Figure 8).

### REFERENCES CITED

[1] H. Zhu, J. F. Klemic, S. Chang, P. Bertone, A. Casmayor, K.G. Klemic, D. Smith, M. Gerstein, M. A. Reed, M. Snyder, Nat. Genet 2000, 26, 283-289.
[2] R. M. T. de Wildt, C. R. Mundy, B. D. Gorick, I. M. Tomlinson, Nat, Biotechnol. 2000, 18, 989-994.
[3] U. Reineke, R. Volkmer-Engert, J. Schneider-Mergener, Curr. Opin. Biotechnol. 2001, 12, 59-64.
[4] P. J. Hergenrother, K. M. Depew, S. L. Schreiber, J. Am. Chem. Soc. 2000, 122, 7849-7850.
[5] R. L. McCarley, T. D. McCarley, Anal. Chem. 1997, 69, 130-136.
[6] J. L. Trevor, K. R. Lykke, M. J. Pellin, L. Hanley, Langmuir 1998, 14, 1664-1673.
[7] L. Hanley, O. Kornieko, E. T. Ada, E. Fuoco, J. L. Trevor, J. Mass. Spectrom. 1999, 34, 705-723.
[8] D. J. Graham, D. D. Price, B. D. Ratner, Langmuir 2002, 18, 1518-1527.
[9] T. Shibue, T. Nakanishi, T. Matsuda, T. Asahi, T. Osaka, Langmuir 2002, 18, 1528-1534.
[10] D. Dogruel, P. William, R. W. Nelson, Anal. Biochem. 1995, 67, 4343-4348.
[11] A. H. Brockman, R. Orlando, Anal. Biochem. 1995, 67, 4581-4585.
[12] J. Bundy, C. Fenselau, Anal. Biochem. 1999, 71, 1460-1463.
[13] R. W. Nelson, D. Nedelkov, K. A. Tubbs, Electrophoresis 2000, 21, 1155-1163.
[14] T. Natsume, H. Nakayama, T. Isobe, Trends Biotechnol. 2001, 19(10), S28-S33.
[15] M. Merchant, S. R. Weinberger, Electrophoresis 2000, 21, 1164-1177.
[16] H. Zou, Q. Zhang, Z. Guo, B. Guo, Q. Zhang, X. Chen, Angew. Chem. 2002, 114, 668-670; Angew. Chem., Int. Ed. 2002, 41, 646-648
[17] K. L. Prime, G. M. Whitesides, Science 1991, 252, 1164-1167.
[18] K. L. Prime, G. M. Whitesides, J. Am. Chem. Soc. 1993, 115, 10714-10721.
[19] C. Roberts, C. S. Chen, M. Mrksich, V. Martichonok, D. E. Ingber, G. M. Whitesides, J. Am. Chem. Soc. 1998, 120, 6548-6555.
[20] B. T. Houseman, M. Mrksich, Angew. Chem. 1999, 111, 876-880; Angew. Chem., Int. Ed. 1999, 38(6), 782-785.
[21] M. Mrksich, G. B. Sigal, G. M. Whitesides, Langmuir 1995, 11, 4383-4385.
[22] B. T. Houseman, J. H. Huh, S. J. Kron, M. Mrksich, Na.t Biotechnol. 2002, 20, 270-274.
[24] The majority of the SAMs in this work gave mainly sodium adducts on MALDI spectra. Ions below *mlz* 500 were not characterized due to the numerous background signals from the matrix.
[25] When reporting the ratio of alkanethiolates present in a mixed SAM, the ratio of alkanethiolates in the solutions used for the formation of the monolayer is satted.
[26] H: height of peaks, L: the peak for LacNAc, G: the peak for GlcNAc.
[27] B. T. Houseman, M. Mrksich, Chem. Biol. 2002, 9, 443-454.
[28] C. D. Hodneland, Y. S. Lee, D. H. Min, M. Mrksich, Proc. Natl. Acad Sci. U. S. A. 2002, 99(8), 5084-5052.

## Claims

1. A method of characterizing the immobilization of a biomolecule on a self-assembled monolayer (SAM) on a biochip comprising the steps of:
contacting the SAM that is capable of covalently binding the biomolecule with a solution comprising the biomolecule,
rinsing the SAM to remove the solution;
optionally applying a matrix; and
analyzing the SAM with matrix-assisted laser desorption / ionization - time of flight mass spectrometry (MALDI-TOF MS) to determine the presence of immobilized biomolecule.

2. The method of claim 1 , in which the SAM comprises immobilized molecule capable of binding the biomolecule.

3. The method of claim 2, wherein the immobilized molecule capable of binding the biomolecule is a carbohydrate ligand, or is a peptide.

4. The method of claim 2, wherein the SAM further comprises oligo(ethylene glycol)-terminated alkanethiol.

5. A method of characterizing the enzymatic modification of an immobilized ligand using a self-assembled monolayer (SAM) on a biochip and matrix-assisted laser desorption / ionization and time of flight mass spectrometry (MALDI-TOF MS) comprising the steps of:
providing a SAM that presents an immobilized ligand capable of undergoing enzymatic modification;
treating the SAM with a solution containing an enzyme;
rinsing the SAM to remove the solution;
optionally applying a matrix; and
analyzing the SAM by MALDI-TOF MS.

6. A method of obtaining kinetic data for biological interactions using a self-assembled monolayer (SAM) on a biochip and matrix-assisted laser desorption / ionization and time of flight mass spectrometry (MALDI-TOF MS) comprising the steps of:
providing a test SAM that presents an immobilized ligand capable of undergoing an enzymatic modification;
providing a control SAM that presents the immobilized ligand;
treating the test SAM with a solution containing an enzyme for a time sufficient to allow the enzymatic modification;
rinsing the test SAM to remove the solution;
applying matrixes to each of the test and control SAMs;
analyzing each of the test and control SAMs by MALDI-TOF MS; and
comparing the results to obtain kinetic data,

7. The method of claim 6, wherein multiple test SAMs are provided and exposed to the solution comprising the enzyme for differing amounts of time.

8. A method of detecting enzymatic modification of an immobilized ligand on a self-assembled monolayer (SAM) comprising the steps of:
providing a self-assembled monolayer (SAM) which is inert to the non-specific adsorption of biomolecules;
immobilizing a ligand onto the surface of the SAM to form a ligand-immobilized SAM;
reacting the ligand-immobilized SAM with an enzyme for a time sufficient to modify the ligand to obtain a modified ligand-hnmobilized SAM;
rinsing the modified ligand-immobilized SAM to remove the excess solution; and
analyzing the modified ligand-immobilized SAM with matrix-assisted laser desorption ionization-time of flight mass spectrometry (MALDI-TOF MS).

9. The method of claim 8, further comprising optionally applying a matrix to the modified ligand-immobilized SAM prior to the analyzing step.

10. The method of claim 8 wherein the SAM comprises oligo(ethylene glycol)-terminated alkanethiol groups, mannitol-terminated alkanethiol groups, diene-terminated alkanethiol groups, or dieneophile-terminated alkanethiol groups.

11. The method of claim 8, wherein immobilizing the ligand onto the surface of the inert SAM comprises, contacting the surface of the inert SAM with the ligand and a second enzyme which catalyzes formation of a covalent bond between the ligand and the surface.

12. The method of claim 10, wherein immobilizing the ligand unto the surface of the inert SAM comprises, contacting the diene-terminated alkanethiol groups with a dieneophile or a diene.

13. A method of detecting the enzymatic modification of a ligand comprising the steps of:
reacting a ligand with a first enzyme in solution for a time sufficient to obtain a modified ligand;
contacting the solution containing the modified ligand with the surface of a SAM, which is inert to the non-specific adsorption of biomolecules, for a time sufficient to obtain a modified ligand-immobilized SAM;
rinsing the modified ligand-immobilized SAM to remove the excess solution; and
analyzing the modified ligand-immobilized SAM with matrix-assisted laser desorption/ionization-time of flight mass spectrometry (MALDI-TOF MS).

14. The method of claim 13, further comprising, prior to the contacting step, activating the SAM.

15. The method of claim 14, wherein activating comprises electrically, photochemically, enzymatically, or chemically modifying the SAM to make it capable of reacting with the ligand.

16. A biochip for use in MALDI-TOF MS comprising:
a SAM, which is inert to the non-specific adsorption of biomolecules, and a matrix.

17. The biochip of claim 16, wherein the SAM comprises oligo(ethylene glycol)-temiinated alkanethiol groups, or mannitol-terminated alkanethiol groups.

18. The biochip of claim 16, wherein the SAM comprises an immobilized ligand, optionally an enzyme-modified ligand.

19. A biochip for use in MALDI-TOF MS, comprising:
a SAM, which is inert to the non-specific adsorption of biomolecules, and
a layer overlying the SAM which comprising a plurality of openings.

20. The biochip of claim 19, wherein the overlying layer contains 96 openings.

21. The biochip of claim 20, wherein the overlying layer comprises a polymeric material.

22. The biochip of claim 20, wherein the layer overlying the SAM is removable from the biochip, the layer overlapping the SAM being optionally attached to the biochip with a releasing agent.

23. An enzymatic assay kit comprising:
a biochip for use in MALDI-TOF MS, comprising an immobilized ligand-modified SAM, which is inert to the non-specific adsorption of biomolecules, and a layer overlying the SAM which comprising a plurality of openings; and an enzymatic solution capable of modifying the immobilized ligand-modified SAM.
